# EUROPÄISCHE PATENTSCHRIFT

(11) **EP 2 522 291 B1**
(45) Veröffentlichungstag und Bekanntmachung des Hinweises auf die Patenterteilung: **11.12.2013**
(21) Anmeldenummer: 12003421.0
(22) Anmeldetag: 04.05.2012
(51) Int. Cl.: A61B 18/00, A61B 18/14, A61B 17/29

(54) **Elektrochirurgisches Instrument**
Electro-surgical instrument
Instrument électrochirurgical

(30) Priorität: 13.05.2011 DE 102011075781
(43) Veröffentlichungstag der Anmeldung: 14.11.2012
(73) Patentinhaber: Karl Storz GmbH & Co. KG, 78532 Tuttlingen (DE)
(72) Erfinder: Emmerich, Bernd, 78532 Tuttlingen (DE)

(56) Entgegenhaltungen:
- EP-A1- 0 795 301
- EP-A1- 1 681 027
- EP-A1- 2 042 113
- US-A- 5 810 805
- US-B1- 6 273 887

## Beschreibung

Die vorliegende Erfindung bezieht sich auf ein elektrochirurgisches Instrument.

In der Elektrochirurgie bzw. bei elektrochirurgischen Maßnahmen wird durch Stromfluss und aufgrund des elektrischen Widerstands von Gewebe (Joulesche) Wärme im Gewebe erzeugt. Durch Form und Anordnung von dabei verwendeten Elektroden wird der Stromfluss möglichst genau lokalisiert. Durch die entstehende Wärme wird das stromdurchflossene Gewebe verödet bzw. zerstört. Dadurch können Gewebe beispielsweise verklebt oder verschlossen und Blutungen gestillt werden.

In der Regel werden in der Elektrochirurgie hochfrequente Wechselströme verwendet, um eine Stimulation von Nerven und andere unerwünschte Nebenwirkungen zu vermeiden. Die Begriffe "Elektrochirurgie" und "HF-Chirurgie" werden deshalb oft synonym verwendet. Ein weiterer, oft synonym verwendeter Begriff ist der der Elektrokauterisation.

Bei mikroinvasiven und anderen Eingiffen werden elektrochirurgische Verfahren angewendet, um großflächig Gewebe zu veröden, um auf kleinem Raum Gefäße zu verschließen ßen oder um Schnittflächen bereits beim Schneiden oder unmittelbar danach zu veröden. Für diese und andere Anwendungen sind jeweils unterschiedliche Stromdichteverteilungen im Gewebe vorteilhaft und erwünscht. Medizinisches Personal, insbesondere Ärztinnen und Ärzte, können die Stromdichteverteilung durch Verwendung unterschiedlich geformter Elektroden bzw. Werkzeuge und durch die Art der Verwendung dieser Elektroden beeinflussen.

Die US6,273,887B1 offenbart ein elektrochirurgisches Instrument mit zwei Maulteilen, wobei eines der Maulteile um seine Längsachse rotiert werden kann. Auf diese Weise ist einmal eine größere und einmal eine kleinere Elektrodenfläche des Maulteils dem zweiten Maulteil zugewandt.

Vergleichbare Instrumente sind auch in der EP0795301A1, US5,810805 und EP1681027A1 beschrieben.

Die aus dem Stand der Technik bekannten Instrumente haben den Nachteil, dass die Elektrodenflächen des rotierbaren Maulteils nicht unabhängig voneinander elektrisch kontaktiert werden können.

Eine Aufgabe der vorliegenden Erfindung besteht darin, ein verbessertes elektrochirurgisches Instrument bereitzustellen.

Diese Aufgabe wird durch die Gegenstände der unabhängigen Ansprüche gelöst.

Weiterbildungen sind in den abhängigen Ansprüchen angegeben.

Ein elektrochirurgisches Instrument umfasst ein erstes Maulteil mit einem ersten Elektrodenbereich und einem zweiten Elektrodenbereich und ein zweites Maulteil, wobei zumindest entweder das erste Maulteil oder das zweite Maulteil um eine Schwenkachse so schwenkbar ist, dass die Maulteile einander angenähert und voneinander entfernt werden können, wobei das erste Maulteil relativ zu dem zweiten Maulteil um eine Rotationsachse zwischen einer ersten vorbestimmten Arbeitsposition und einer zweiten vorbestimmten Arbeitsposition rotierbar ist, wobei in der ersten Arbeitsposition der erste Elektrodenbereich des ersten Maulteils dem zweiten Maulteil zugewandt ist, und wobei in der zweiten Arbeitsposition der zweite Elektrodenbereich des ersten Maulteils dem zweiten Maulteil zugewandet ist.

Das elektrochirurgische Instrument ist ein Instrument für elektrochirurgische Maßnahmen im oben beschriebenen Sinn, insbesondere zur Elektrokauterisation. Das zweite Maulteil kann ein elektrisch isolierendes Material oder eine elektrisch isolierende Oberfläche aufweisen. Alternativ oder zusätzlich kann das zweite Maulteil ein elektrisch leitfähiges Material oder elektrisch leitfähige Abschnitte aufweisen, die Elektroden bilden, und deren offenliegende Oberflächenbereiche Elektroderibereiche bzw. Elektrodenflächenbereiche bilden. Wenn das zweite Maulteil eine oder mehrere Elektroden und eine oder mehrere Elektrodenbereiche aufweist, können diese gegenüber den Elektrodenbereichen des ersten Maulteils elektrisch isoliert oder mit diesen elektrisch leitfähig verbunden sein.

Die Schwenkachse, um die zumindest entweder das erste Maulteil oder das zweite Maulteil schwenkbar ist, ist insbesondere zur Längsachse des elektrochirurgischen Instruments senkrecht. Ein mechanisch besonders einfacher und robuster Aufbau kann realisierbar sein, wenn nur das zweite Maulteil um eine Schwenkachse senkrecht zur Längsachse des elektrochirurgischen Instruments schwenkbar ist, während das erste Maulteil nur um die Rotationsachse rotierbar ist. Alternativ können beide Maulteile um je eine Schwenkachse schwenkbar sein, wobei die Schwenkachsen der Maulteile insbesondere parallel zueinander und senkrecht zur Rotationsachse und senkrecht zur Längsachse des elektrochirurgischen Instruments sind.

Die Rotationsachse des ersten Maulteils ist insbesondere parallel zur Längsachse des ersten Maulteils bzw. parallel oder im Wesentlichen parallel zur Richtung der größten linearen Ausdehnung des ersten Maulteils. Die Rotationsachse des ersten Maulteils ist damit insbesondere parallel zur Längsachse des elektrochirurgischen Instruments. Wenn das erste Maulteil um eine Schwenkachse schwenkbar ist, ist die Rotationsachse des ersten Maulteils insbesondere in der Position des ersten Maulteils, in der es dem zweiten Maulteil am nächsten ist, parallel zur Längsachse des elektrochirurgischen Instruments.

Die erste vorbestimme Arbeitsposition und die zweite vorbestimmte Arbeitsposition können durch eine oder mehrere Rasteinrichtungen am distalen Ende des elektrochirurgischen Instruments und/oder am proximalen Ende des elektrochirurgischen Instruments (lösbar) festgelegt sein. Alternativ oder zusätzlich können Markierungen am elektrochirurgischen Instrument vorgesehen sein, die für medizinisches Personal erkennbar machen, ob das erste Maulteil sich in der ersten vorbestimmten Arbeitsposition oder in der zweiten vorbestimmen Arbeitsposition befindet. Die Markierungen sind insbesondere am proximalen Ende des elektrochirurgischen Instruments angeordnet.

Die erste vorbestimmte Arbeitsposition und die zweite vorbestimme Arbeitsposition sind insbesondere durch einen vorbestimmten Winkel voneinander beabstandet. Zwischen der ersten vorbestimmten Arbeitsposition und der zweiten vorbestimmten Arbeitsposition und/oder außerhalb des durch die beiden vorbestimmten Arbeitspositionen bestimmten Winkelbereichs können eine oder mehrere weitere Arbeitspositionen vorgesehen sein. Alternativ oder zusätzlich zu diskreten vorbestimmten Arbeitspositionen können eine oder mehrere Arbeitsbereiche vorgesehen sein, die jeweils ein Kontinuum von Arbeitspositionen umfassen.

Der erste Elektrodenbereich und der zweite Elektrodenbereich sind voneinander elektrisch isoliert und werden durch zwei verschiedene Elektroden bzw. zwei verschiedene elektrisch leitfähige Bauteile gebildet.

Insbesondere unterscheidet sich die Gestalt des ersten Elektrodenbereichs von der Gestalt des zweiten Elektrodenbereichs.

Die Rotation des ersten Maulteils zwischen der ersten vorbestimmten Arbeitsposition und der zweiten vorbestimmten Arbeitsposition kann eine Anpassung der Gestalt der jeweils aktiven bzw. dem zweiten Maulteil zugewandten Elektrodenfläche an unterschiedliche Gegebenheiten, unterschiedliche Verwendungen bzw. unterschiedliche elektrochirurgische Maßnahmen und die dabei erwünschten unterschiedlichen Stromdichteverteilungen innerhalb sehr kurzer Zeit ermöglichen. Medizinisches Personal muss deshalb in vielen Fällen für unterschiedliche elektrochirurgische Maßnahmen nicht mehr das elektrochirurgische Instrument austauschen, sondern lediglich das erste Maulteil rotieren.

Bei einem elektrochirurgischem Instrument, wie es hier beschrieben ist, weist insbesondere der erste Elektrodenbereich eine stärkere Krümmung auf als der zweite Elektrodenbereich.

Beispielsweise weist der erste Elektrodenbereich einen streifen- oder linienförmigen Bereich hoher Krümmung auf, der eine stark lokalisierte Stromdichteverteilung ermöglicht. Insbesondere ist der erste Elektrodenbereich derart gekrümmt, dass eine Kontur eines Querschnitts entlang einer Ebene senkrecht zur Längsrichtung des ersten Maulteils zumindest abschnittsweise einen kleinen Krümmungsradius aufweist. Insbesondere weist der erste Elektrodenbereich eine scharfe Kante auf. Die Kante kann als Schneidkante ausgebildet sein, die gleichzeitig ein Schneiden und eine Elektrokauterisation der Schnittkanten ermöglicht.

Der zweite Elektrodenbereich weist beispielsweise eine geringe Krümmung auf oder ist eben oder im Wesentlichen eben. Mit dieser geometrischen Gestalt ermöglicht der zweite Elektrodenbereich eine Stromdichteverteilung, die in einem verhältnismäßig großen Raumbereich im Wesentlichen konstant ist. Damit ist beispielsweise eine großflächige Elektrokauterisation möglich, insbesondere ein elektrochirurgischer Verschluss eines Gefäßes ohne dessen Durchtrennung.

Bei einem elektrochirurgischen Instrument, wie es hier beschrieben ist, sind der erste Elektrodenbereich durch eine erste Elektrode und der zweite Elektrodenbereich durch eine zweite Elektrode gebildet, wobei die erste Elektrode und die zweite Elektrode voneinander elektrisch isoliert sind.

Die erste Elektrode und die zweite Elektrode sind insbesondere mittels eines keramischen oder Kunststoff-Bauteils elektrisch voneinander isoliert. Die elektrische Isolation der beiden Elektroden und damit der beiden Elektrodenbereiche ermöglicht eine selektive Aktivierung bzw. Einbeziehung in einen Stromkreis nur einer der beiden Elektroden und nur eines der beiden Elektrodenbereiche. Eine elektrochirurgische Wirkung der jeweils anderen Elektroden bzw. des jeweils anderen Elektrodenbereichs ist dadurch zumindest weitgehend vermeidbar.

Ein elektrochirurgisches Instrument, wie es hier beschrieben ist, umfasst ferner eine elektrische Kontakteinrichtung zur elektrischen Kontaktierung lediglich der ersten Elektrode, wenn das erste Maulteil sich in der ersten Arbeitsposition befindet und zur elektrischen Kontaktierung lediglich der zweiten Elektrode, wenn das erste Maulteil sich in der zweiten Arbeitsposition befindet.

Die Kontakteinrichtung umfasst insbesondere einen Gleitkontakt bzw. Schleifkontakt, der abhängig von der Position des ersten Maulteils eine elektrisch leitfähige Verbindung entweder zu einem mit der ersten Elektrode elektrisch leitfähig verbundenen oder einstückig ausgebildeten elektrisch leitfähigen Bauteil oder zu einem mit der zweiten Elektrode elektrisch leitfähig verbundenen oder einstückig ausgebildeten elektrisch leitfähigen Bauteil bildet. Dies ermöglicht eine automatische selektive Kontaktierung bzw. elektrische Aktivierung bzw. Einbindung in den elektrischen Schaltkreis desjenigen Elektrodenbereichs, der dem zweiten Maulteil momentan gegenüberliegt. Somit ist insbesondere ein separater Schaltvorgang oder eine andere separate Manipulation an einem elektrischen Schaltkreis überflüssig. Dies kann die Handhabung des elektrochirurgischen Instruments vereinfachen.

Die Kontakteinrichtung ist insbesondere ausgebildet, um mittelbar oder unmittelbar eine elektrisch leitfähige Verbindung zwischen einem Steckkontakt oder einer anderen elektrischen Anschlusseinrichtung und der ersten Elektrode oder der zweiten Elektrode zu bilden. Der Steckkontakt oder die andere elektrische Anschlusseinrichtung ist insbesondere zur elektrisch leitfähigen Verbindung mit einer elektrischen Leistungsquelle mittels eines Kabels ausgebildet.

Die Kontakteinrichtung ist beispielsweise am distalen oder am proximalen Ende des elektrochirurgischen Instruments angeordnet. Aufgrund des für eine Kontakteinrichtung erforderlichen Bauraums kann eine Anordnung am proximalen Ende des elektrochirurgischen Instruments vorteilhaft sein.

Bei einem elektrochirurgischen Instrument, wie es hier beschrieben ist, kann ferner das zweite Maulteil einen Elektrodenbereich aufweisen, der dem ersten Maulteil zugewandt ist.

Das zweite Maulteil kann mehrere Elektrodenbereiche aufweisen, die elektrisch leitfähig miteinander verbunden (insbesondere durch dieselbe Elektrode bzw. dasselbe elektrisch leitfähige Bauteil gebildet) oder voneinander elektrisch isoliert sein können. Der Elektrodenbereich am zweiten Maulteil ist insbesondere von den Elektrodenbereichen am ersten Maulteil elektrisch isoliert bzw. mit diesen nicht unmittelbar elektrisch leitfähig verbunden. Vielmehr sind der Elektrodenbereich am zweiten Maulteil einerseits und zumindest der dem zweiten Maulteil momentan zugewandte Elektrodenbereich am ersten Maulteil andererseits ausgebildet, um mit zwei verschiedenen Polen einer elektrischen Leistungsquelle verbunden zu werden. Anders ausgedrückt ist das elektrochirurgische Instrument insbesondere für eine bipolare Verwendung ausgebildet, bei der ein (insbesondere hochfrequenter Wechsel-)Strom zwischen dem Elektrodenbereich am zweiten Maulteil und dem momentan aktiven bzw. dem zweiten Maulteil zugewandten Elektrodenbereich am ersten Maulteil erzeugbar ist. Bei einer bipolaren Verwendung des elektrochirurgischen Instruments kann eine räumlich besonders gut definierte Stromdichteverteilung und eine somit besonders gut lokalisierte elektrochirurgische Wirkung erzeugt werden.

Alternativ ist das elektrochirurgische Instrument mit oder ohne eine Elektrodenfläche am zweiten Maulteil für eine ausschließliche oder wahlweise monopolare Verwendung vorgesehen und ausgebildet. Bei einer monopolaren Verwendung des elektrochirurgischen Instruments kann der Stromkreis über eine großflächige Elektrode am Körper des Patienten, die auch als passive Elektrode bezeichnet wird, geschlossen werden.

Bei einem elektrochirurgischen Instrument mit einem Elektrodenbereich am zweiten Maulteil kann ein vom ersten Mauteil abgewandter Oberflächenbereich des zweiten Maulteils elektrisch isolierend ausgebildet sein. Dadurch kann ein unerwünschter bzw. parasitärer Stromfluss in Bereichen außerhalb des Zwischenraums zwischen den Maulteilen reduziert oder unterbunden werden.

Bei einem elektrochirurgischen Instrument mit einem Elektrodenbereich am zweiten Maulteil ist der Elektrodenbereich am zweiten Maulteil insbesondere zumindest abschnittsweise eben oder zumindest abschnittsweise konkav ausgebildet. Beispielsweise weist der Elektrodenbereich am zweiten Maulteil eine Nut bzw. eine Kerbe mit V-förmigem oder U-förmigem oder trapezförmigem oder anderem Querschnitt auf. Das elektrochirurgische Instrument kann so ausgebildet sein, dass ein stegförmiger oder schneidenförmiger Abschnitt eines Elektrodenbereichs am ersten Maulteil in die Nut bzw. die Kerbe am zweiten Maulteil eingreifen kann, wenn die beiden Maulteile ihre nächst zueinander benachbarten Positionen einnehmen.

Ein elektrochirurgisches Instrument, wie es hier beschrieben ist, umfasst insbesondere einen Schaft, an dessen distalem Ende die Maulteile angeordnet sind, eine Handhabungseinrichtung mit einer Rotationseinrichtung am proximalen Ende des Schafts, wobei die Rotationseinrichtung zur manuellen Betätigung ausgebildet ist, und eine Übertragungseinrichtung, welche die Rotationseinrichtung mit dem ersten Maulteil mechanisch koppelt, zur Übertragung zumindest entweder eines Drehmoments oder einer Kraft von der Rotationseinrichtung zum ersten Maulteil, um das erste Maulteil zwischen seiner ersten Arbeitsposition und seiner zweiten Arbeitsposition zu rotieren.

Die Rotationseinrichtung umfasst insbesondere ein Drehrad, das durch medizinisches Personal manuell gedreht werden kann. Die Übertragungseinrichtung umfasst insbesondere eine Welle zum Übertragen eines Drehmoments. Die Rotationseinrichtung und die Übertragungseinrichtung ermöglichen eine Auswahl bzw. einen Wechsel bzw. eine Einstellung der Arbeitsposition des ersten Maulteils vom proximalen Ende des elektrochirurgischen Instruments aus. Das distale Ende des elektrochinugischen Instruments mit den beiden Maulteilen muss deshalb nicht manuell erreichbar sein, um zwischen Arbeitspositionen des ersten Maulteils zu wechseln. Insbesondere wenn das elektrochirurgische Instrument mit einem langen (geraden oder gekrümmten; starren oder flexiblen) Schaft für mikroinvasive Anwendungen ausgebildet ist, können die Rotationseinrichtung und die Übertragungseinrichtung einen schnellen Wechsel zwischen unterschiedlichen Elektrodenbereichen und unterschiedlichen Stromdichteverteilungen ermöglichen. Dabei kann das distale Ende des elektrochirurgischen Instruments beispielsweise in einem Hohlraum verbleiben, in dem ein Eingriff mittels des elektrochirurgischen Instruments stattfindet.

Bei einem elektrochirurgischen Instrument mit einer Rotationseinrichtung und einer Übertragungseinrichtung kann ferner eine Rastfunktion vorgesehen sein, um das erste Maulteil in den Arbeitspositionen jeweils rastend zu arretieren. Die Rastfunktion kann einerseits medizinischem Personal eine taktile Rückmeldung über das Erreichen einer Arbeitsposition bereitstellen. Andererseits kann die Rastfunktion eine unerwünschte Rotation des ersten Maulteils aus einer Arbeitsposition heraus verhindern, indem sie dieser Rotation ein Widerstandsmoment entgegensetzt. Beides kann die Ergonomie und die Sicherheit der Handhabung des elektrochirurgischen Instruments verbessern.

Alternativ oder zusätzlich zu einer Rotationseinrichtung können ein elektrischer Hohlwellenmotor, ein Ultraschallmotor, ein auf dem Memory- bzw. Gedächtnis-Effekt bei einem pseudoelastischen Material beruhender Antrieb oder ein anderer motorischer Antrieb vorgesehen sein, um das erste Maulteil zwischen den vorbestimmten Arbeitspositionen zu rotieren. Einer der beschriebenen Antriebe kann gleichzeitig dazu ausgebildet sein, um das erste Maulteil in einer ausgewählten vorbestimmten Arbeitsposition zu halten. Wenn der Antrieb am distalen Ende des elektrochirurgischen Instruments vorgesehen ist, kann ferner auf die Übertragungseinrichtung zum Übertragen eines Drehmoments oder einer Kraft zum ersten Maulteil verzichtet werden. Eine motorische Rotation des ersten Maulteils zwischen den vorbestimmten Arbeitspositionen kann eine Auswahl der Arbeitsposition und damit des aktiven bzw. dem zweiten Maulteil zugewandten Elektrodenbereichs durch Fußschalter oder -taster, mittels Spracheingabe, aufgrund von Gesten oder abhängig von einer Phase in einem Workflow bzw. einem Arbeitsablauf ermöglichen.

Ein elektrochirurgisches Instrument, wie es hier beschrieben ist, kann so ausgebildet sein, dass das erste Maulteil nach distal abnehmbar ist.

Wenn das elektrochirurgische Instrument einen Schaft aufweist, an dessen distalem Ende die Maulteile angeordnet sind, ist das erste Maulteil insbesondere durch eine Bewegung parallel zur Längsachse des Schafts bzw. zur lokalen Längsachse des Schafts an dessen distalem Ende nach distal abnehmbar. Die Abnehmbarkeit des ersten Maulteils kann eine Reinigung und Sterilisierung des elektrochirurgischen Instruments vereinfachen.

Bei einem elektrochirurgischen Instrument mit einer Übertragungseinrichtung, wie sie hier beschrieben ist, ist insbesondere das erste Maulteil zusammen mit der Übertragungseinrichtung nach distal aus dem Schaft entnehmbar.

Die gemeinsame Entnehmbarkeit des ersten Maulteils und der Übertragungseinrichtung ermöglicht eine dauerhafte und damit robuste mechanische Verbindung des Maulteils und der Übertragungseinrichtung. Die Entnehmbarkeit nach distal kann eine Reinigung des elektrochirurgischen Instruments vereinfachen, indem - im Gegensatz zu einer Entnehmbarkeit in entgegengesetzter Richtung - Verunreinigungen am ersten Maulteil nicht in den Schaft eingebracht werden.

Ein elektrochirurgisches Instrument, bei dem das erste Maulteil zusammen mit der Übertragungseinrichtung nach distal aus dem Schaft entnehmbar ist, kann ferner eine Rastfunktion zur Arretierung des proximalen Endes der Übertragungseinrichtung an der Handhabungseinrichtung umfassen. Die Rastfunktion ist insbesondere ausgebildet, um das proximale Ende der Übertragungseinrichtung lösbar mit der Rotationseinrichtung zu verbinden. Die Rastfunktion an der Verbindung zwischen dem proximalen Ende der Übertragungseinrichtung und der Rotationseinrichtung vorzusehen, vermeidet Probleme hinsichtlich des Bauraums, die beispielsweise am distalen Ende des elektrochirurgischen Instruments limitierend wirken würden. Ferner kann bei der genannten Anordnung der Rastfunktion eine Dearretierung in Bereiche einer Handhabungseinrichtung am proximalen Ende des elektrochirurgischen Instruments vorgesehen sein.

Bei einem elektrochirurgischen Instrument, wie es hier beschrieben ist, kann zumindest entweder der erste Elektrodenbereich des ersten Maulteils oder der zweite Elektrodenbereich des ersten Maulteils oder ein dem ersten Maulteil zugewandter Oberflächenbereich des zweiten Maulteils in der Nähe der Schwenkachse in Längsrichtung konkav ausgebildet sein.

Im Fall einer in Längsrichtung konkaven Ausbildung des zweiten Maulteils nahe der Schwenkachse ist insbesondere ein Elektrodenbereich am zweiten Maulteil in Längsrichtung konkav ausgebildet. Eine in Längsrichtung konkave Ausbildung ist insbesondere gegeben, wenn ein Schnitt entlang einer zur Schwenkachse senkrechten Ebene eine konkave Kontur des jeweiligen Maulteils enthält. Eine konkave Ausbildung zumindest eines Maulteils nahe der Schwenkachse kann - insbesondere bei einer bipolaren Ausgestaltung des elektrochirurgischen Instruments - beim Schließen bzw. Aufeinanderzubewegen der Maulteile eine übermäßig hohe Stromdichte, im Extremfall einen Kurzschluss, zwischen Elektrodenflächen an den Maulteilen vermindern oder verhindern.

Bei einem Verfahren zum Vorbereiten einer elektrochirurgischen Maßnahme mittels eines elektrochirurgischen Instruments mit einem ersten Maulteil und einem zweiten Maulteil, wobei zumindest entweder das erste Maulteil oder das zweite Maulteil um eine Schwenkachse so schwenkbar ist, dass dabei die Maulteile einander angenähert oder voneinander entfernt werden können, wird das erste Maulteil in eine erste Arbeitsposition, in der ein erster Elektrodenbereich des ersten Maulteils dem zweiten Maulteil zugewandt ist oder in eine zweite Arbeitsposition, in der ein zweiter Elektrodenbereich des ersten Maulteils dem zweiten Maulteil zugewandet ist, rotiert.

Insbesondere wenn der erste Elektrodenbereich und der zweite Elektrodenbereich unterschiedliche Gestalt aufweisen und somit für unterschiedliche Stromdichteverteilungen ausgebildet sind, kann das Rotieren des ersten Maulteils in eine der vorbestimmten Arbeitspositionen eine schnelle Anpassung der Stromdichteverteilung an unterschiedliche Anwendungen und Anforderungen ermöglichen.

Vor dem beschriebenen Schritt des Rotierens kann medizinisches Personal eine erwünschte Gestalt einer Elektrode bestimmen. Aufgrund der bestimmten erwünschten Gestalt kann in einem nächsten Schritt bestimmt werden, welcher von mehreren Elektrodenbereichen am ersten Maulteil des elektrochirurgischen Instruments der erwünschten Gestalt am nächsten kommt, um dann bei dem beschriebenen Schritt des Rotierens diesen Elektrodenbereich dem zweiten Maulteil gegenüber anzuordnen. Nach dem Rotieren des ersten Maulteils und dem so erfolgten Anpassen des aktiven Elektrodenbereichs an die erwünschte Gestalt kann das elektrochirurgische Instrument bei einer elektrochirurgischen Maßnahme verwendet werden.

Bei einem Verfahren zum Vorbereiten einer elektrochirurgischen Maßnahme, wie sie hier beschrieben ist, wird insbesondere ein elektrochirurgisches Instrument verwendet, wie es hier beschrieben ist.

### Kurzbeschreibung der Figuren

Nachfolgend werden Ausführungsformen anhand der beigefügten Figuren näher erläutert. Es zeigen:
- Figur 1: eine schematische Darstellung eines elektrochirurgischen Instruments;
- Figur 2: eine schematische Schnittdarstellung von Maulteilen;
- Figur 3: eine weitere schematische Schnittdarstellung der Maulteile aus Figur 2;
- Figur 4: eine schematische Schnittdarstellung von weiteren Maulteilen;
- Figur 5: eine weitere schematische Schnittdarstellung der Maulteile aus Figur 4;
- Figur 6: eine schematische Schnittdarstellung von weiteren Maulteilen;
- Figur 7: eine weitere schematische Schnittdarstellung der Maulteile aus Figur 6;
- Figur 8: eine schematische Schnittdarstellung von weiteren Maulteilen;
- Figur 9: eine weitere schematische Schnittdarstellung der Maulteile aus Figur 8;
- Figur 10: eine schematische Schnittdarstellung von weiteren Maulteilen;
- Figur 11: eine weitere schematische Schnittdarstellung der Maulteile aus Figur 10;
- Figur 12: eine schematische Schnittdarstellung von weiteren Maulteilen;
- Figur 13: eine weitere schematische Schnittdarstellung der Maulteile aus Figur 12;
- Figur 14: eine schematische Schnittdarstellung des Instruments aus Figur 1;
- Figur 15: eine weitere schematische Schnittdarstellung des Instruments aus den Figuren 1 und 14;
- Figur 16: eine schematische Schnittdarstellung eines schwenkbaren Teils einer Handhabungseinrichtung;
- Figur 17: eine weitere schematische Schnittdarstellung des schwenkbaren Teils aus Figur 16;
- Figur 18: eine schematische Schnittdarstellung eines weiteren schwenkbaren Teils einer Handhabungseinrichtung;
- Figur 19: ein schematisches Flussdiagramm.

### Beschreibung der Ausführungsformen

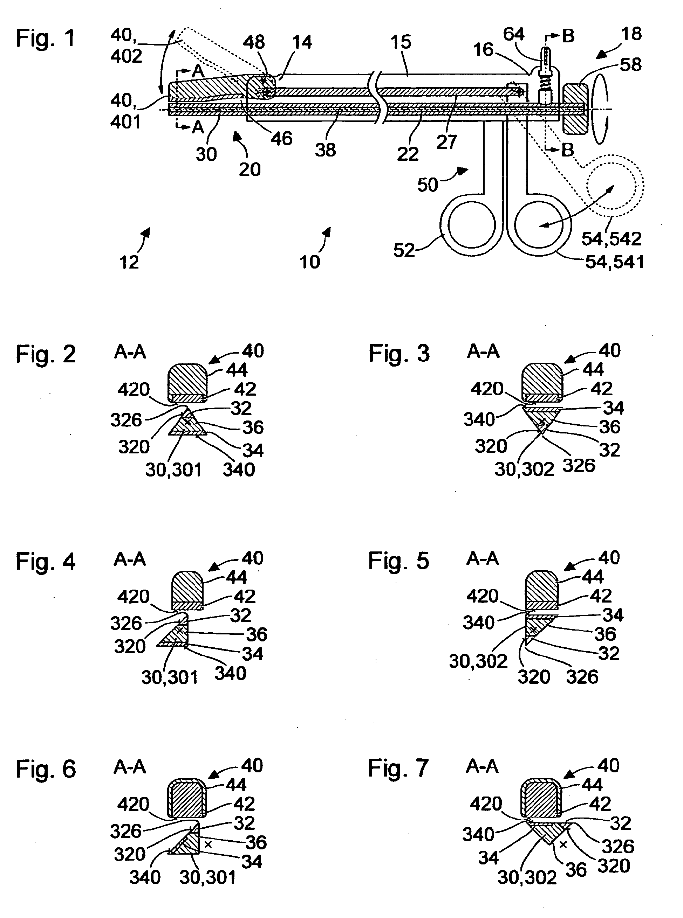

Figur 1 zeigt eine schematische Darstellung eines elektrochirurgischen Instruments 10. Figur 1 weist teilweise den Charakter einer schematischen Schnittdarstellung auf. Einige Schnittflächen sind als schraffierte Flächen dargestellt. Dabei sind elektrisch leitfähige Bereiche von links unten nach rechts oben und elektrisch isolierende Bereiche von links oben nach rechts unten schraffiert. Andere Bereiche und Merkmale des elektrochirurgischen Instruments sind lediglich in Konturen angedeutet. Diese Bereiche und Merkmale haben für die im Folgenden beschriebene Funktionalität in der Regel eine geringere Bedeutung. In Figur 1 sind ferner die Positionen zweier Schnittebenen A-A und B-B dargestellt. Schnitte entlang den Schnittebenen A-A und B-B sind in den Figuren 2 bis 13 bzw. 14 und 15 dargestellt.

Das elektrochirurgische Instruments 10 umfasst ein distales Ende 12 und ein proximales Ende 18. Das elektrochirurgische Instrument 10 umfasst einen Schaft 15 mit einem distalen Ende 14 und einem proximalen Ende 16. Der Schaft kann ein elektrisch isolierendes Material oder eine elektrisch isolierende Beschichtung an seiner Außenseite aufweisen. Ein Werkzeug 20 ist am distalen Ende 12 des elektrochirurgischen Instruments 10 angeordnet und mit dem distalen Ende 14 des Schafts 15 permanent oder zerstörungsfrei (mit oder ohne Verwendung von Werkzeug) lösbar verbunden. Das Werkzeug 20 umfasst ein erstes Maulteil 30 und ein zweites Maulteil 40, das um eine Schwenkachse 48 schwenkbar ist. Die Schwenkachse 48 ist senkrecht zur Zeichenebene der Figur 1 und zur Längsachse des elektrochirurgischen Instruments 10, insbesondere des Schafts 15.

Das zweite Maulteil 40 ist in Figur 1 in einer ersten, geschlossenen Position 401 und - in gestrichelten Linien - in einer zweiten, geöffneten Position 402 dargestellt. In der ersten, geschlossenen Position 401 berühren die Maulteile 30, 40 einander zumindest abschnittsweise oder - wie in Figur 1 angedeutet - liegen einander zumindest in einem Bereich mit einem geringen und insbesondere konstanten Abstand gegenüber.

In einem Bereich 46 nahe der Schwenkachse 48 ist die dem ersten Maulteil 30 gegenüberliegende Oberfläche des zweiten Maulteils 40 leicht konkav ausgebildet. Insbesondere ist die dem ersten Maulteil 30 gegenüberliegende Oberfläche des zweiten Maulteils 40 im Bereich 46 in Längsrichtung konkav ausgebildet, so dass der dargestellte Schnitt entlang einer Ebene parallel zur Längsachse des elektrochirurgischen Instruments 10 und parallel zur Richtung der maximalen linearen Ausdehnung des zweiten Maulteils 40 eine konkave Kontur aufweist. Der konkave Bereich 46 kann bei bipolarer Verwendung des elektrochirurgischen Instruments 10 beim Schließen der Maulteile 30, 40 die Stromdichte nahe der Schwenkachse 48 des zweiten Maulteils 40 reduzieren.

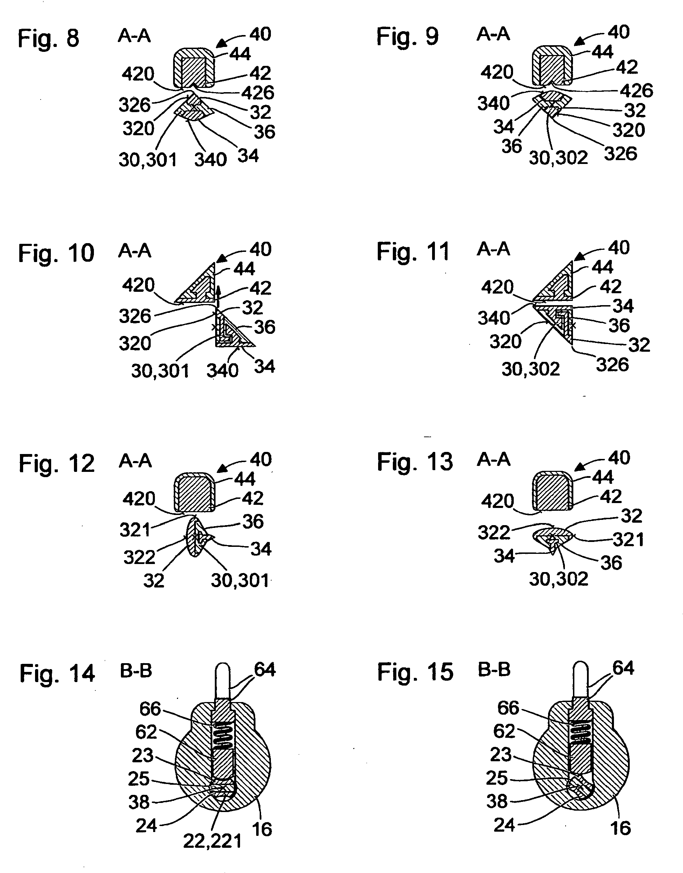

Im Schaft 15 sind eine Welle 22 und eine Zugstange 27 als Übertragungseinrichtungen angeordnet, die sich jeweils vom distalen Ende 14 bis zum proximalen Ende 16 des Schafts 15 oder darüber hinaus erstrecken. Die Welle 22 und die Zugstange 27 sind jeweils insbesondere elektrisch leitfähig ausgebildet. Zwischen der Welle 22 und der Zugstange 27 kann eine Wand zur elektrischen Isolierung vorgesehen sein, die in Figur 1 nicht dargestellt ist. Die Welle 22 ist am distalen Ende 14 des Schafts 15 mit dem ersten Maulteil 30 mechanisch gekoppelt oder, wie in Figur 1 angedeutet, einstückig ausgebildet. Die Welle 22 ist um eine Rotationsachse 38 rotierbar. Details eines Aufbaus der Welle 22 mit mehreren elektrisch leitfähigen Bereichen und einem dazwischenliegenden Isolierbereich sowie ein in Figur 1 nur angedeuteter Steckkontakt 64 sind unten mit Bezug auf die Figuren 14 und 15 dargestellt. Ein weiterer, in Figur 1 nicht dargestellter Steckkontakt kann zur Kontaktierung des proximalen Endes der Zugstange 27 vorgesehen sein.

Die Zugstange 27 ist am distalen Ende 14 des Schafts 15 so mit dem zweiten Maulteil 40 gelenkig gekoppelt, dass eine Verschiebung der Zugstange 27 in Längsrichtung des Schafts 15 mit einer Schwenkbewegung des zweiten Maulteils 40 um die Schwenkachse 48 verbunden ist. Am proximalen Ende 18 weist das elektrochirurgische Instrument 10 eine Handhabungseinrichtung 50 mit einem feststehenden Teil 52 und einem schwenkbaren Teil 54 auf. Der feststehende Teil 52 und der schwenkbare Teil 54 der Handhabungseinrichtung 50 weisen jeweils einen Bügel oder eine Öse auf, in den bzw. die ein oder mehrere Finger eingeführt werden können.

Der schwenkbare Teil 54 der Handhabungseinrichtung 50 ist zwischen eine ersten Position 541 und einer zweiten Position 542 schwenkbar, die in Figur 1 nur in gestrichelten Linien dargestellt ist. Der schwenkbare Teil 54 der Handhabungseinrichtung 50 ist so mit dem proximalen Ende der Zugstange 27 gekoppelt, dass ein Schwenken des schwenkbaren Teils 54 zwischen den Positionen 541, 542 eine Verschiebung der Zugstange 27 in Längsrichtung des Schafts 15 und damit ein Schwenken des zweiten Maulteils 40 um dessen Schwenkachse 48 bewirkt. Insbesondere befindet sich das zweite Maulteil 40 in seiner ersten, geschlossenen Position 401, wenn sich der schwenkbare Teil 54 der Handhabungseinrichtung 50 in seiner ersten Position 541 befindet, und in der zweiten, offenen Position 402, wenn sich der schwenkbare Teil 54 der Handhabungseinrichtung 50 in seiner zweiten Position 542 befindet.

Die Handhabungseinrichtung 50 am proximalen Ende 18 des elektrochirurgischen Instruments 10 umfasst ferner ein Drehrad 58, das mit dem proximalen Ende der Welle 22 mechanisch gekoppelt, insbesondere mechanisch starr verbunden ist. Das Drehrad 58, insbesondere dessen Mantelfläche weist zur Vereinfachung einer manuellen Betätigung insbesondere eine Riffelung oder eine andere Struktur auf, die in der Schnittdarstellung der Figur 1 nicht erkennbar ist. Eine Rotation des Drehrads 58 um die Achse 38 hat eine entsprechende Rotation der Welle 22 und des ersten Maulteils 30 um dieselbe Achse 38 zur Folge.

Zur rastenden, lösbaren Arretierung des Drehrads 58, der Welle 22 und des ersten Maulteils 30 in vorbestimmten Winkelpositionen ist eine Einrichtung vorgesehen, die in Figur 1 lediglich angedeutet und unten mit Bezug auf die Figuren 14 und 15 ausführlicher dargestellt ist.

Anders als in Figur 1 dargestellt kann der Schaft 15 von der Handhabungseinrichtung abnehmbar ausgebildet sein. Insbesondere ist das proximale Ende 16 des Schafts 15 in eine Ausnehmung in der Handhabungseinrichtung 50 einführbar und rastend arretierbar. Zum Lösen der rastenden Arretierung ist beispielsweise ein Druckknopf an der Handhabungseinrichtung 50 vorgesehen.

Die Figuren 2 bis 13 zeigen schematische Schnittdarstellungen verschiedener Ausführungsformen der Maulteile 30, 40. Dargestellt sind jeweils Schnitte entlang der in Figur 1 angedeuteten Ebenen A-A senkrecht zur Zeichenebene der Figur 1, senkrecht zur Rotationsachse 38 des ersten Maulteils 30 und senkrecht zur Längsachse des Schafts 15 des elektrochirurgischen Instruments 10. Jeweils zwei Figuren mit aufeinanderfolgenden Nummern zeigen das erste Maulteil in zwei verschiedenen Arbeitspositionen 301, 302, wobei das erste Maulteil durch Rotation um die Rotationsachse 38 (vgl. Figur 1) um einen vorbestimmten Winkel von der einen Arbeitsposition in die andere überführt werden kann. Die zur dargestellten Schnittebene A-A senkrechte Rotationsachse ist in den Figuren 2 bis 13 jeweils als "x" bzw. als liegendes Kreuz angedeutet. Der vorbestimmte Winkel beträgt bei den meisten Beispielen 180 Grad, bei einem Beispiel (Figuren 6, 7) 45 Grad und bei einem Beispiel (Figuren 12, 13) 90 Grad.

Die Figuren 2 bis 13 zeigen sechs verschiedene Ausführungsformen des ersten Maulteils 30 und fast ebenso viele verschiedene Ausführungsformen des zweiten Maulteils 40. Die Ausführungsformen des ersten Maulteils 30 und die Ausführungsformen des zweiten Maulteils 40 können teilweise auch anders als in den Figuren 2 bis 13 gezeigt kombiniert werden. Ausnahmen ergeben sich aus besonderen Funktionen, die ggf. nachfolgend erwähnt sind.

Die Figuren 2 und 3 zeigen ein erstes Maulteil 30 mit einem gleichschenklig-dreieckigen Querschnitt. Ein Eck des Querschnitts wird von einer ersten Elektrode 32 mit einer dachförmigen Elektrodenfläche 320 und einer Kante 326 gebildet. Die dachförmige Elektrodenflächen 320, insbesondere die Kante 326, ist bei der in Figur 2 gezeigten ersten Arbeitsposition 301 des ersten Maulteils 30 dem zweiten Maulteil 40 zugewandt und bei der in Figur 3 gezeigten zweiten Arbeitsposition 302 vom zweiten Maulteil 40 abgewandt.

Eine von dem durch die erste Elektrode 32 gebildeten Eck des Querschnitts abgewandt gegenüberliegende Seite des Querschnitts des ersten Maulteils 30 wird von einer zweiten Elektrode 34 mit einer im Wesentlichen ebenen Elektrodenfläche 340 gebildet. Da die zweite Elektrode 34 auch die beiden angrenzenden Ecken des dreieckigen Querschnitts des ersten Maulteils 30 bildet, weist die Elektrodenfläche 340 an den Rändern eines großen ebenen Bereichs je eine Kante und daran anschließend je einen schmalen streifenförmigen Bereich auf. Die zweite Elektrode 34 und der große ebene Bereich der Elektrodenfläche 340 der zweiten Elektrode 34 sind bei der in Figur 2 gezeigten ersten Arbeitsposition 301 des ersten Maulteils 30 vom zweiten Maulteil 40 abgewandt und bei der in Figur 3 gezeigten zweiten Arbeitsposition 302 des ersten Maulteils 30 dem zweiten Maulteil 40 zugewandt.

Das in den Figuren 2 und 3 gezeigte zweite Maulteil 40 umfasst eine Elektrode 42 mit einem rechteckigen Querschnitt und einer ebenen Elektrodenfläche 420. Ferner umfasst das zweite Maulteil 40 eine Isolierung 44 in Form eines Isolierkörpers, der drei Seiten des Querschnitts der Elektrode 42 umgreift. Außenliegende Kanten der Isolierung 44 sind abgerundet. Die Isolierung 44 weist beispielsweise einen elektrisch isolierenden Kunststoff, eine elektrisch isolierende Keramik oder ein anderes elektrisch isolierendes Material auf.

Bei der in Figur 2 gezeigten ersten Arbeitsposition 301 des ersten Maulteils 30 entsteht zwischen der dachförmigen Elektrodenfläche 320 bzw. der Kante 326 am ersten Maulteil 30 und der gegenüberliegenden ebenen Elektrodenfläche 420 am zweiten Maulteil 40 eine Stromdichteverteilung, die von einer starken Konzentration und einem ausgeprägten Maximum des Betrags der Stromdichte in der Nähe der Kante 326 am ersten Maulteil 30 dominiert wird.

Die dachförmige Elektrodenfläche 320 und die Kante 326 sind im Zusammenwirken mit der Elektrodenfläche 420 am zweiten Maulteil 40 geeignet, Gewebe mechanisch zu quetschen und unter Umständen zu durchtrennen bzw. zu schneiden. Dabei bewirkt die durch die Form der Elektrodenflächen 320, 420 hervorgerufene Stromdichteverteilung eine verhältnismäßig kleinräumige Joulsche Erwärmung und Verödung des Gewebes und kann ferner den Schneidevorgang unterstützen.

Bei der in Figur 3 gezeigten zweiten Arbeitsposition 302 des ersten Maulteils 30 liegen mit der im Wesentlichen ebenen Elektrodenfläche 340 der zweiten Elektrode 36 des ersten Maulteils 30 und der ebenen Elektrodenfläche 420 der Elektrode 42 des zweiten Maulteils 40 zwei ausgedehnte Elektrodenflächen, dievon den Rändern abgesehen keine Struktur aufweisen, einander im Wesentlichen parallel gegenüber. Es stellt sich eine in einem großen Raumbereich im Wesentlichen homogene Stromdichte ohne ausgeprägtes Maximum oder ausgeprägte Konzentration ein. Diese Stromdichteverteilung eignet sich für eine großräumige Joulesche Erwärmung und Verödung von Gewebe, wobei das Gewebe gleichzeitig nicht oder allenfalls geringfügig mechanisch bearbeitet wird.

Die Figuren 4 und 5 zeigen ein erstes Maulteil 30, das ähnlich wie beim Ausführungsbeispiel der Figuren 2 und 3 einen dreieckigen, jedoch keinen gleichschenklig-dreieckigen Querschnitt aufweist. Ähnlich wie beim Ausführungsbeispiel der Figuren 2 und 3 stellt sich bei der in Figur 4 dargestellten ersten Arbeitsposition des ersten Maulteils 30 eine stark lokalisierte Stromdichteverteilung mit ausgeprägtem Maximum in der Umgebung der Kante 326 und bei der in Figur 5 dargestellten zweiten Arbeitsposition 302 des ersten Maulteils 30 eine großräumig im Wesentlichen homogene Stromdichteverteilung ohne ausgeprägte Konzentration bzw. ausgeprägtes Maximum ein. Mögliche Anwendungen entsprechen den oben anhand der Figuren 2 und 3 dargestellten Anwendungen.

Bei der in Figur 4 gezeigten ersten Arbeitsposition 301 des ersten Maulteils 30 kann die asymmetrische Gestalt der dachförmigen Elektrodenfläche 320 der ersten Elektrode 32 des ersten Maulteils 30 eine asymmetrische Stromdichteverteilung bewirken. Insbesondere kann sich zwischen der in Figur 4 nach links orientierten Flanke der dachförmigen Elektrodenfläche 320 der ersten Elektrode 32 des ersten Maulteils 30 und der Elektrode 42 des zweiten Maulteils 40 eine höhere Stromdichte einstellen als im rechts gegenüberliegenden Raumbereich zwischen der in Figur 4 nach rechts orientierten Flanke der dachförmigen Elektrodenfläche 320 der ersten Elektrode 32 des ersten Maulteils 30 und der Elektrodenfläche 420 der Elektrode 42 des zweiten Maulteils 40. Das Ausführungsbeispiel der Figuren 4 und 5 kann deshalb geeignet sein, in der in Figur 4 dargestellten ersten Arbeitsposition 301 des ersten Maulteils 30 beispielsweise Gefäße zu durchschneiden, wobei eines der beiden entstehende Gefäßenden in stärkerem Maße durch Elektrokauterisation verschlossen wird als das andere.

Das Ausführungsbeispiel der Figuren 4 und 5 unterscheidet sich vom Ausführungsbeispiel der Figuren 2 und 3 ferner dadurch, dass der die Isolierung 44 bildende Isolierkörper die Elektrode 42 des zweiten Maulteils 40 nicht an drei Seiten umgreift, sondern nur an einer Seite der Elektrode 42 angrenzt. Die Elektrodenfläche 420 des zweiten Maulteils 40 weist deshalb - ähnlich wie die Elektrodenfläche 340 der zweiten Elektrode 34 des ersten Maulteils 30 des Ausführungsbeispiels der Figuren 2 und 3 - an den Rändern eines großen ebenen Bereichs Kanten und an diese angrenzend je einen schmalen streifenförmigen Bereich auf.

Die Figuren 6 und 7 zeigen ein Ausführungsbeispiel, bei dem das erste Maulteil 30 ähnlich wie beim Ausführungsbeispiel der Figuren 4 und 5 einen asymmetrisch-dreieckigen bzw. nicht gleichschenklig-dreieckigen Querschnitt aufweist. Im Gegensatz zu den Ausführungsbeispielen der Figuren 2 bis 5 ist beim Ausführungsbeispiel der Figuren 6 und 7 die zweite Elektrode 34 nicht an dem Rand des Querschnitts angeordnet, der dem durch die erste Elektrode 32 gebildeten Eck gegenüberliegt, sondern an einer an dieses Eck angrenzenden Seite. Eine Folge ist, dass der Abstand zwischen der Elektrodenfläche 320 der ersten Elektrode 32 einerseits und der Elektrodenfläche 340 der zweiten Elektrode 34 andererseits kleiner ist als bei den Ausführungsbeispielen der Figuren 2 bis 5. Eine weitere Folge ist, dass zwischen den in den Figuren 6 und 7 dargestellten Arbeitspositionen 301, 302 des ersten Maulteils 30 eine Rotation von nur ca. 45 Grad liegt. In einer weiteren, nicht dargestellten Arbeitsposition kann eine weitere, in den Figuren 6 und 7 nicht dargestellte Elektrodenfläche am ersten Maulteil 30 aktiviert bzw. dem zweiten Maulteil 40 gegenüber angeordnet sein.

Das Ausführungsbeispiel der Figuren 6 und 7 unterscheidet sich von den Ausführungsbeispielen der Figuren 2 bis 5 ferner dadurch, dass der Querschnitt der Elektrode 42 des zweiten Maulteils 40 einen wesentlich größeren Flächeninhalt aufweist und die Isolierung 44 in Form einer Isolierschicht die Elektrode 42 des zweiten Maulteils 40 an drei Seiten umgibt. Insbesondere bei einer Ausgestaltung der Elektrode 42 aus Metall kann das Maulteil 40 aufgrund des größeren Querschnitts der Elektrode 42 ein höheres Biegemoment aufnehmen, auch wenn die Isolierung 44 ein Material mit geringer Festigkeit aufweist.

Die Figuren 8 und 9 zeigen ein Ausführungsbeispiel, bei dem das erste Maulteil einen Querschnitt mit der Gestalt eines Drachenvierecks mit einer abgerundeten Ecke aufweist. Eine erste Elektrode 32 des ersten Maulteils 30 bildet eine dachförmige Elektrodenfläche mit einer Kante 326. Eine zweite Elektrode 34 mit einer konvex gewölbten und glatten Elektrodenfläche 340 bildet das abgerundete Eck des Querschnitts, das dem durch die erste Elektrode 32 gebildeten Eck gegenüberliegt.

Bei dem in den Figuren 8 und 9 dargestellten Beispiel erstreckt sich weder die dachförmige Elektrodenfläche 320 der ersten Elektrode 32 noch die gekrümmte glatte Elektrodenfläche 340 der zweiten Elektrode 34 bis zu einem der beiden anderen Ecken des Querschnitts des ersten Maulteils 30. Diese beiden anderen Ecken des Querschnitts des ersten Maulteils 30 werden somit durch die Isolierung 36 zwischen den Elektrodenfläche 32, 34 gebildet.

Der Querschnitt des zweiten Maulteils 40 des Ausführungsbeispiels der Figuren 8 und 9 ähnelt in einigen Merkmalen den Ausführungsbeispielen der Figuren 2 bis 7, insbesondere dem Ausführungsbeispiel der Figuren 6 und 7. Im Unterschied zu den Ausführungsbeispielen der Figuren 2 bis 7 weist die Elektrodenfläche 420 der Elektrode 42 des zweiten Maulteils 40 eine Nut bzw. Kerbe 426 mit einem V-förmigen Querschnitt auf.

Bei der in Figur 8 gezeigten ersten Arbeitsposition 301 des ersten Maulteils 30 liegt die Kante 326 der ersten Elektrode 32 des ersten Maulteils 30 der Nut 426 in der Elektrode 42 des zweiten Maulteils 40 gegenüber. Das elektrochirurgische Instrument kann so ausgebildet sein, dass die Kante 326 am ersten Maulteil 30 in die Nut 426 am zweiten Maulteil 40 teilweise oder vollständig eingreifen kann. Bei der in Figur 9 dargestellten zweiten Arbeitsposition 302 des ersten Maulteils 30 liegt die gewölbte glatte Elektrodenfläche 340 der zweiten Elektrode 34 des ersten Maulteils 30 der Elektrodenfläche 420 der Elektrode 42 des zweiten Maulteils 40 gegenüber.

Ähnlich wie bei den Ausführungsbeispielen der Figuren 2 bis 7 bewirken beim Ausführungsbeispiel der Figuren 8 und 9 die unterschiedlichen Gestalten der Elektrodenflächen 320, 340 bei der ersten Arbeitsposition 301 des ersten Maulteils 30 eine starke Konzentration des Stromflusses mit einem ausgeprägten Maximum der Stromdichte in der Umgebung der Kante 326 an der ersten Elektrode 32 und in der zweiten Arbeitsposition 302 des ersten Maulteils 30 die glatte Gestalt der Elektrodenfläche 340 eine deutlich weniger konzentrierte Stromdichteverteilung. Der Unterschied zwischen den Stromdichteverteilungen in den Arbeitspositionen 301, 302 des ersten Maulteils 30 ist beim Ausführungsbeispiel der Figuren 8 und 9 jedoch geringer als bei den Ausführungsbeispielen der Figuren 2 bis 7, da die zur Kante 326 an der ersten Elektrode 32 des ersten Maulteils 30 korrespondierende Nut 426 an der Elektrode 42 des zweiten Maulteils 40 der Konzentration der Stromdichte teilweise entgegenwirkt. Ferner bewirkt die konvexe Krümmung der Elektrodenfläche 340 der zweiten Elektrode 34 des ersten Maulteils 30 und die konvexen Kanten beiderseits der Nut 426 der Elektrode 42 des zweiten Maulteils 40 eine Konzentration der Stromdichte in deren Umgebung und deshalb ein deutlich weniger homogene Stromdichteverteilung als bei den Ausführungsbeispielen der Figuren 3 bis 7 in der jeweils zweiten Arbeitsposition 302 des ersten Maulteils 30.

Die Figuren 10 und 11 zeigen ein Ausführungsbeispiels, das in einigen Merkmalen den Ausführungsbeispielen der Figuren 2 bis 9 ähnelt. Beim Ausführungsbeispiel der Figuren 10 und 11 weisen beide Maulteile 30, 40 jeweils einen dreieckigen Querschnitt auf. Ein Eck des dreieckigen Querschnitts des ersten Maulteils 30 wird durch eine erste Elektrode 32 bzw. deren Kante 326 gebildet. Die gegenüberliegende Seite des dreieckigen Querschnitts des ersten Maulteils 30 wird durch eine im Wesentlichen ebene Elektrodenfläche 340 einer zweiten Elektrode 34 gebildet.

Da bei dem dargestellten Beispiel die zweite Elektrode 34 die gesamte Seite des dreieckigen Querschnitts des ersten Maulteils 30 bildet, weist die Elektrodenfläche 340 an den Rändern eines großen ebenen Bereichs je eine Kante und an diese anschließend zwei schmale streifenförmige Bereiche, die sich in die angrenzenden Seiten hinein erstrecken, auf. Zwischen den Elektroden 32, 34 des ersten Maulteils 30 ist eine Isolierung angeordnet, auf deren räumliche Gestalt unten eingegangen wird.

Das zweite Maulteil 40 weist an seiner dem ersten Maulteil 30 zugewandten Seite eine Elektrode 42 mit einer im Wesentlichen ebenen Elektrodenfläche 420 auf. Da die Elektrode 42 sich über die gesamte Seite des dreieckigen Querschnitts des zweiten Maulteils 40 erstreckt, weist die Elektrodenfläche 420 an zwei gegenüberliegenden Rändern eines großen ebenen Bereichs je eine Kante und an diese anschließend je einen schmalen streifenförmigen Bereich, der in die jeweils angrenzende Seite des dreieckigen Querschnitts hineinreicht, auf. Von diesen schmalen streifenförmigen Bereichen der Elektrodenfläche 420 abgesehen, werden zwei Seiten des dreieckigen Querschnitts des zweiten Maulteils 40 durch eine Isolierung 44 gebildet.

Bei der in Figur 10 dargestellten ersten Arbeitsposition 301 des ersten Maulteils 30 liegen die dachförmige Elektrodenfläche 320, insbesondere deren Kante 326, der ersten Elektrode 32 des ersten Maulteils 30 und ein Rand der im Wesentlichen ebenen Elektrodenfläche 420 der Elektrode 42 des zweiten Maulteils 40 einander gegenüber. Das erste Maulteil 30 kann einem in Figur 10 dargestellten Pfeil entsprechend am zweiten Maulteil 40 vorbei bewegt werden, um Gewebe durch Scherung mechanisch zu schneiden.

Bei der in Figur 11 dargestellten zweiten Arbeitsposition 302 des ersten Maulteils 30 liegen die im Wesentlichen ebene Elektrodenfläche 340 der zweiten Elektrode 34 des ersten Maulteils 30 und die im Wesentlichen ebene Elektrodenfläche 420 der Elektrode 42 des zweiten Maulteils 40 einander parallel oder im Wesentlichen parallel gegenüber.

Die in den beiden Arbeitspositionen des ersten Maulteils 30 bewirkten Stromdichteverteilungen entsprechen näherungsweise den Stromdichteverteilungen bei den Ausführungsbeispielen der Figuren 2 bis 7, insbesondere den Stromdichteverteilungen des Ausführungsbeispiels der Figuren 4 und 5.

Bei den Ausführungsbeispielen der Figuren 2 bis 9 sind die Elektroden 32, 34 bzw. 42 und die als Isolierkörper oder Isolierschicht ausgebildete Isolierung 36 bzw. 44 jeweils insbesondere stoffschlüssig gefügt. In den Figuren 10 und 11 sind Querschnitte der Elektroden 32, 34, 42 sowie der Isolierungen 36, 44 mit Hinterschneidungen erkennbar. Dies ermöglicht alternativ oder zusätzlich zur stoffschlüssigen eine formschlüssige Verbindung.

Die Figuren 12 und 13 zeigen ein Ausführungsbeispiel das in einigen Merkmalen den Ausführungsbeispielen der Figuren 2 bis 11 ähnelt. Insbesondere ähnelt das zweite Maulteil 40 dem zweiten Maulteil des Ausführungsbeispiels der Figuren 6 und 7.

Im Unterschied zu den Ausführungsbeispielen der Figuren 2 bis 11 weist beim Ausführungsbeispiel der Figuren 12 und 13 das erste Maulteil 30 einen Querschnitt mit einem Rand mit einem elliptischen Abschnitt auf. Der elliptische Abschnitt des Rands des Querschnitts des ersten Maulteils 30 wird durch eine Elektrodenfläche 320 einer ersten Elektrode 32 des ersten Maulteils 30 gebildet.

Bei der in Figur 12 dargestellten ersten Arbeitsposition 301 des ersten Maulteils 30 liegt ein stark gekrümmter erster Elektrodenbereich 321 der Elektrodenfläche 320 der ersten Elektrode 32 nahe einem Brennpunkt der Ellipse dem zweiten Maulteil 40 gegenüber. Bei der in Figur 13 dargestellten zweiten Arbeitsposition 302 des ersten Maulteils 30 liegt ein schwach gekrümmter zweiter Elektrodenbereich 322 der Elektrodenfläche 320 der ersten Elektrode 32 des ersten Maulteils 30 dem zweiten Maulteil 40 gegenüber. Dadurch stellen sich in den Arbeitspositionen 301, 302 des ersten Maulteils 30 unterschiedliche Stromdichteverteilungen zwischen der Elektrode 32 des ersten Maulteils 30 und der Elektrode 42 des zweiten Maulteils 40 ein. Bei der in Figur 12 gezeigten ersten Arbeitsposition 301 des ersten Maulteils 30 ist der Stromfluss stärker lokalisiert bzw. weist die Stromdichteverteilung ein ausgeprägteres Maximum auf als bei der in Figur 13 dargestellten zweiten Arbeitsposition 302 des ersten Maulteils 30.

Aufgrund der kontinuierlich variierenden Krümmung der Elektrodenfläche 320 der ersten Elektrode 32 am ersten Maulteil 30 können in weiteren Positionen des ersten Maulteils 30 zwischen den in den Figuren 12 und 13 dargestellten Arbeitspositionen 301, 302 Stromdichteverteilungen erzeugt werden, die zwischen denen der Arbeitspositionen 301, 302 liegen.

Das erste Maulteil 30 weist ferner eine zweite Elektrode 34 mit einer Elektrodenfläche 340 mit einer Kante auf. In einer weiteren, in den Figuren 12 und 13 nicht dargestellten und gegenüber der in der Figur 13 dargestellten zweiten Arbeitsposition 302 um 180 Grad gedrehten Arbeitsposition liegen die Elektrodenfläche 340 und die Kante der zweiten Elektrode 34 des ersten Maulteils 30 der Elektrodenfläche 420 der Elektrode 42 des zweiten Maulteils 40 gegenüber. Dabei kann sich eine Stromdichteverteilung einstellen, die den Stromdichteverteilungen bei den Ausführungsbeispielen der Figuren 2 bis 7 jeweils in der ersten Arbeitsposition 301 des ersten Maulteils 30 ähnelt. Die Kante der zweiten Elektrode 34 des ersten Maulteils 30 kann auch zum mechanischen Schneiden ausgebildet sein.

Die Figuren 14 und 1 zeigen schematische Schnittdarstellungen des elektrochirurgischen Instruments 10 aus Figur 1 entlang der in Figur 1 angedeuteten Ebene B-B senkrecht zur Zeichenebene der Figur 1. Im proximalen Ende 16 des Schafts 15 bzw. des elektrochirurgischen Instruments 10 ist die Welle 22 um die Rotationsachse 38 rotierbar gelagert. Die Welle 22 umfasst einen ersten elektrisch leitfähigen Bereich 23, einen zweiten elektrisch leitfähigen Bereich 24 und einen zwischen den elektrisch leitfähigen Bereichen 23, 24 angeordneten Isolierbereich 25, der die elektrisch leitfähigen Bereiche 23, 24 voneinander elektrisch isoliert. Der erste elektrisch leitfähige Bereich 23 der Welle 22 ist mit der ersten Elektrode 32 des ersten Maulteils 30 (vgl. die Figuren 2 bis 13) elektrisch leitfähig verbunden, insbesondere einstückig ausgebildet. Der zweite elektrisch leitfähige Bereich 24 der Welle 22 ist mit der zweiten Elektrode 34 des ersten Maulteils 30 elektrisch leitfähig verbunden, insbesondere einstückig ausgebildet. Der Isolierbereich 25 der Welle 22 kann mit der Isolierung 36 des ersten Maulteils 30 einstückig ausgebildet sein.

Die Welle 22 weist einen Querschnitt mit einer abschnittsweise kreisförmigen Kontur auf. An zwei gegenüberliegenden Stellen weicht die Kontur des Querschnitts der Welle 22 von der Kreisform ab. Insbesondere weist jeder elektrisch leitfähige Bereich 23, 24 der Welle 22 einen konkaven Oberflächenabschnitt auf.

Das elektrochirurgische Instrument weist einen Gleitkontakt 62, einen Steckkontakt 64 und eine Druckfeder zwischen dem Gleitkontakt 62 und dem Steckkontakt 64 auf. Der Gleitkontakt 62 ist in einer Richtung parallel zur Schnittebene B-B und senkrecht zur Rotationsachse 38 verschiebbar im proximalen Ende 16 des Schafts gelagert. Der Steckkontakt 64 ist starr im proximalen Ende 16 befestigt, beispielsweise form- und/oder stoffschlüssig gefügt. Ein vom elektrochirurgischen Instrument abstehendes Ende des Steckkontakts 64 ist zur elektrisch leitfähigen Verbindung mit einem Steckverbinder eines elektrischen Kabels ausgebildet. Dazu weist der Steckkontakt 64 insbesondere einen Schlitz auf, der bei dem in den Figuren 1, 14 und 15 parallel zur Schnittebene B-B (vgl. auch Figur 1) ist. Der Schlitz ermöglicht eine Federelastizität des Steckkontakts 64.

Zwischen dem Steckkontakt 64 und dem Gleitkontakt 62 ist eine Druckfeder 66 in Form einer Schraubenfeder oder ein anderes elektrisch leitfähiges elastisches Element angeordnet. Die Druckfeder 66 bildet eine elektrisch leitfähige Verbindung zwischen dem Steckkontakt 64 und dem Gleitkontakt 62. Ferner drückt die Druckfeder 66 den Gleitkontakt 62 gegen die Welle 22. Bei der in Figur 14 gezeigten Position der Welle 22 liegt der Gleitkontakt 62 in einer Vertiefung des ersten elektrisch leitfähigen Bereichs 23. Damit ist eine elektrisch leitfähige Verbindung zwischen dem Steckkontakt 64 und der ersten Elektrode 32 des ersten Maulteils 30 hergestellt. Durch Formschluss zwischen dem Gleitkontakt 62 und der Vertiefung im ersten elektrisch leitfähigen Bereich 23 der Welle 22 wird die Welle 22 in der in Figur 14 dargestellten ersten Arbeitsposition 221 gehalten. Beim Drehen der Welle 22 aus der ersten Arbeitsposition 221 heraus, beispielsweise in die in Figur 15 gezeigte Position, wird der Gleitkontakt 62 gegen die Kraft der Druckfeder 66 verschoben. Dazu ist ein Mindest-Drehmoment erforderlich, das beispielsweise manuell am Drehrad 58 (vgl. Figur 1) erzeugt werden kann.

Bei der anhand der Figuren 14 und 15 dargestellten Ausführungsform erfüllen der Gleitkontakt 62 und die Druckfeder 66 zwei Funktionen gleichzeitig, nämlich die elektrische Kontaktierung und die rastende Arretierung. Alternativ ist eine Verwirklichung beider Funktionen mittels teilweise oder vollständig separater Einrichtungen möglich.

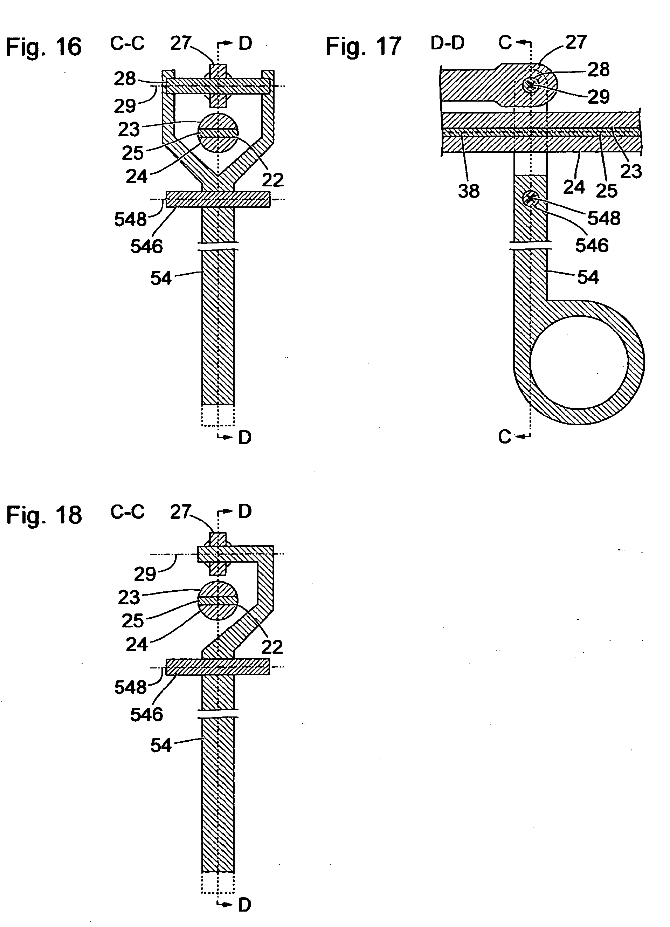

Der schwenkbare Teil 54 der Handhabungseinrichtung 50 ist in Figur 1 nur angedeutet. Die Figuren 16 und 17 zeigen schematische und etwas detailliertere Darstellungen einer Ausführungsform eines schwenkbaren Teils 54 einer Handhabungseinrichtung. Dabei zeigen Figur 16 einen Schnitt entlang einer Ebene C-C senkrecht zur Rotationssachse 38 (vgl. Figur 1) und parallel zu den Ebenen A-A und B-B und Figur 17 einen Schnitt entlang einer Ebene D-D senkrecht zur Ebene C-C und parallel zur Zeichenebene der Figur 1. Die Ebene D-D ist in Figur 16 angedeutet, die Eben C-C ist in Figur 17 angedeutet.

Der schwenkbare Teil 54 der Handhabungseinrichtung weist eine Y-förmige Gestalt auf und ist mittels einer Welle 546 um eine Schwenkachse 548 schwenkbar gelagert. Der schwenkbare Teil 54 ist relativ zur Welle 546 rotierbar auf dieser gelagert, und/oder die Welle 546 ist im elektrochirurgischen Instrument 10 rotierbar gelagert. Zwei Enden des schwenkbaren Teils 54 sind durch eine Welle 28 verbunden, an der das proximale Ende der Zugstange 27 gelagert ist. Die Zugstange 27 ist relativ zum schwenkbaren Teil 54 der Handhabungseinrichtung um eine Schwenkachse 29 schwenkbar gelagert. Die Welle 28 ist relativ zum schwenkbaren Teil 54 der Handhabungseinrichtung rotierbar, und/oder die Zugstange 27 ist relativ zur Welle 28 rotierbar.

Die Welle 28 und die beiden Schenkel des schwenkbaren Teils 54 der Handhabungseinrichtung umschließen einen Raum, in dem die Welle 22 aus zwei elektrisch leitfähigen Bereichen 23, 24 und einem dazwischenliegenden Isolierbereich 25 angeordnet und um die Rotationsachse 38 rotierbar ist. Der schwenkbare Teil 54 der Handhabungseinrichtung ist innerhalb eines vorbestimmten Winkelbereichs, wie er bereits oben anhand der Figur 1 dargestellt ist, schwenkbar. Aufgrund der Kopplung der Zugstange 27 mit dem schwenkbaren Teil 54 der Handhabungseinrichtung mittels der Welle 28 bewirkt jede Schwenkbewegung des schwenkbaren Teils 54 der Handhabungseinrichtung eine Verschiebung der Zugstange 27 und umgekehrt.

Figur 18 zeigt eine schematische Darstellung einer alternativen Ausführungsform des schwenkbaren Teils 54 einer Handhabungseinrichtung, die in einigen Merkmalen dem schwenkbaren Teil 54 aus den Figuren 16 und 17 ähnelt. Dabei zeigt Figur 18 einen Schnitt entlang einer Ebene C-C senkrecht zur Rotationssachse 38 (vgl. Figur 1) und parallel zu den Ebenen A-A und B-B. Ein Schnitt entlang der in Figur 18 angedeuteten Ebene D-D senkrecht zur Ebene C-C und parallel zur Zeichenebene der Figur 1 ähnelt so weitgehend dem in Figur 17 dargestellten Schnitt, dass dieser Schnitt nicht separat dargestellt ist.

Die in Figur 18 dargestellte Ausführungsform des schwenkbaren Teils 54 der Handhabungseinrichtung unterscheidet sich von dem oben anhand der Figuren 16 und 17 dargestellten schwenkbaren Teil insbesondere dadurch, dass es keine Y-förmige, sondern eine näherungsweise L-förmige Gestalt aufweist. Ferner ist die Zugstange 27 rotierbar an einem bolzenförmigen Bereich 548 des schwenkbaren Teils 54 gelagert.

Die anhand der Figur 18 dargestellte Gestalt des schwenkbaren Teils 54 kann einen geringeren Bauraum erfordern als die anhand der Figuren 16 und 17 dargestellte Gestalt. Ferner kann ein seitliches Entnehmen der Welle 22 und/oder des schwenkbaren Teils 54 der Handhabungseinrichtung durch eine Verschiebung parallel zur Ebene C-C möglich sein.

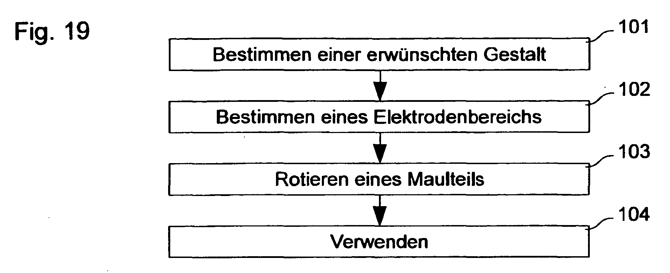

Figur 19 zeigt ein schematisches Flussdiagramm eines Verfahrens, in dessen Rahmen eine elektrochirurgische Maßnahme durchgeführt werden kann, und bei dem ein elektrochirurgisches Instrument, wie es oben anhand der Figuren 1 bis 18 dargestellt ist, verwendbar ist. Obwohl das Verfahren auch mit elektrochirurgischen Instrumenten ausführbar ist, die sich von den oben anhand der Figuren 1 bis 18 dargestellten Ausführungsbeispielen unterscheiden, sind nachfolgend beispielhaft Bezugszeichen aus den Figuren 1 bis 18 verwendet, um das Verständnis zu vereinfachen.

Bei einem ersten Schritt 101 wird durch medizinisches Personal, insbesondere durch eine Ärztin oder einen Arzt, eine erwünschte Gestalt einer Elektrodenfläche, die bei einer nachfolgend durchzuführenden elektrochirurgischen Maßnahme einzusetzen ist, bestimmt. Der erste Schritt 101 kann auf Überlegungen zur erwünschten Stromdichteverteilung und/oder auf praktischen Erfahrungen beruhen und davon abhängig sein, ob beispielsweise großflächig oder großräumig Gewebe lediglich zu veröden oder gleichzeitig zu schneiden ist. Die Entscheidung über die erwünschte Gestalt der Elektrode kann ferner vom erwünschten therapeutischen Erfolg und von der Art des Gewebes abhängen.

Bei einem zweiten Schritt 102 wird bestimmt, welcher Elektrodenbereich 320, 321, 322, 340 an einem rotierbaren ersten Maulteil 30 der erwünschten Gestalt am nächsten kommt. Insbesondere wird bestimmt, ob die Gestalt eines ersten Elektrodenbereichs 320, 321 oder die Gestalt eines zweiten Elektrodenbereichs 322, 340 an einem rotierbaren ersten Maulteil 30 der erwünschten Gestalt ähnlicher ist. Auch der zweite Schritt 102 bzw. die darin enthaltene Entscheidung wird in der Regel vom gleichen medizinischen Personal durchgeführt wie der erste Schritt 101. Der zweite Schritt 102 enthält jedoch im Gegensatz zum ersten Schritt keine Überlegungen, die sich unmittelbar auf therapeutische Maßnahmen und deren Gestaltung beziehen, und auch sonst keine fachlich-medizinischen Überlegungen.

Bei einem dritten Schritt 103 wird das erste Maulteil 30 in eine erste vorbestimmte Arbeitsposition 301, in der ein erster Elektrodenbereich 320; 321 des ersten Maulteils 30 einem zweiten Maulteil 40 zugewandt ist, oder in eine zweite vorbestimmte Arbeitsposition 302, in der ein zweiter Elektrodenbereich 340, 322 des ersten Maulteils 30 dem zweiten Maulteil 40 zugewandt ist, rotiert. Ob das erste Maulteil 30 in die erste vorbestimmte Arbeitsposition 301 oder in die zweite vorbestimmte Arbeitsposition 302 rotiert wird, ist insbesondere abhängig vom Ergebnis des zweiten Schritts 102. Ferner kann das Maulteil 30 in eine dritte oder eine weitere vorbestimmte Arbeitsposition rotiert werden.

Der dritte Schritt 103 stellt eine Vorbereitung einer elektrochirurgischen Maßnahme, insbesondere eines dabei zu verwendenden elektrochirurgischen Instruments dar. Der dritte Schritt 103 selbst stellt weder eine chirurgische noch eine therapeutische Maßnahme dar. Zur Vorbereitung der elektrochirurgischen Maßnahme können im weiteren Sinn auch der erste Schritt 101 und insbesondere der zweite Schritt 102 gezählt werden. Die Vorbereitung des elektrochirurgischen Instruments 10 erfolgt jedoch ausschließlich im dritten Schritt 103.

Bei einem vierten Schritt 104 wird das beim dritten Schritt 103 vorbereitete elektrochirurgische Instrument 10 verwendet, um die vorgesehene elektrochirurgische Maßnahme durchzuführen. Nur der vierte Schritt 104 stellt einen chirurgischen und unter Umständen auch einen therapeutischen Schritt dar.

### Bezugszeichen

- 10: elektrochirurgisches Instrument
- 12: distales Ende des elektrochirurgischen Instruments 10
- 14: distales Ende des Schafts 15 des elektrochirurgischen Instruments 10
- 15: Schaft des elektrochirurgischen Instruments 10
- 16: proximales Ende des Schaft 15 des elektrochirurgischen Instruments 10
- 18: proximales Ende des elektrochirurgischen Instruments 10

- 20: Werkzeug am distalen Ende des elektrochirurgischen Instruments 10
- 22: Welle bzw. Übertragungseinrichtung zur Übertragung eines Drehmoments
- 23: erster elektrisch leitfähiger Bereich der ersten Übertragungsstange 22
- 24: zweiter elektrisch leitfähiger Bereich der ersten Übertragungsstange 22
- 25: Isolierbereich zwischen elektrisch leitfähigen Bereichen der ersten Übertragungsstange 22
- 27: Zugstange bzw. Übertragungseinrichtung zur Übertragung einer Zugkraft
- 28: Welle zwischen Zugstange 27 und schwenbarem Teil 54 der Handhabungseinrichtung 50
- 29: Schwenkachse zwischen Zugstange 27 und schwenbarem Teil 54 der Handhabungseinrichtung 50

- 30: erstes Maulteil des Werkzeugs 20
- 301: erstes Maulteil des Werkzeugs 20 in erster Arbeitsposition
- 302: erstes Maulteil des Werkzeugs 20 in zweiter Arbeitsposition
- 32: erste Elektrode am ersten Maulteil 30
- 320: Elektrodenfläche an der ersten Elektrode 32
- 321: erster Elektrodenbereich
- 322: zweiter Elektrodenbereich der ersten Elektrode 32
- 326: Kante an der ersten Elektrode 32
- 34: zweite Elektrode am ersten Maulteil 30
- 340: Elektrodenfläche an der zweiten Elektrode 34
- 346: Kante an der zweiten Elektrode 34
- 36: Isolierung (Isolierkörper, Isolierschicht)
- 38: Rotationsachse des ersten Maulteils 30

- 40: zweites Maulteil des Werkzeugs 20
- 401: zweites Maulteil des Werkzeugs 20 in erster (geschlossener) Position
- 402: zweites Maulteil des Werkzeugs 20 in zweiter (geöffneter) Position
- 42: Elektrode am zweiten Maulteil 40
- 420: Elektrodenfläche der Elektrode 42 am zweiten Maulteil 40
- 426: Nut in der Elektrode 42 am zweiten Maulteil 40
- 44: Isolierung (Isolierkörper, Isolierschicht)
- 46: konkaver Bereich am zweiten Maulteil 40
- 48: Schwenkachse des zweiten Maulteils 24

- 50: Handhabungseinrichtung
- 52: feststehender Teil der Handhabungseinrichtung 50
- 54: schwenkbarer Teil der Handhabungseinrichtung 50
- 541: schwenkbarer Teil 54 in erster Position
- 542: schwenkbarer Teil 54 in zweiter Position
- 546: Welle
- 548: Schwenkachse des schwenbaren Teils 54 der Handhabungseinrichtung 50
- 58: Drehrad

- 62: Gleitkontakt
- 64: Steckkontakt
- 66: Druckfeder

- 101: erster Schritt (Bestimmen einer erwünschten Elektrodenform)
- 102: zweiter Schritt (Bestimmen, welcher Elektrodenbereich)
- 103: dritter Schritt (Rotieren des ersten Maulteils)
- 104: vierter Schritt (Verwenden des elektrochirurgischen Instruments)

## Patentansprüche

1. **Elektrochirurgisches Instrument** (10), mit:
einem **ersten Maulteil** (30) mit einem ersten Elektrodenbereich (320; 321) und einem zweiten Elektrodenbereich (340; 322);
einem **zweiten Maulteil** (40),
wobei zumindest entweder das erste Maulteil (30) oder das zweite Maulteil (40) um eine Schwenkachse (48) so **schwenkbar** ist, dass die Maulteile (30, 40) einander angenähert oder voneinander entfernt werden können,
wobei das erste Maulteil (30) relativ zu dem zweiten Maulteil (40) um eine Rotationsachse (38) zwischen einer **ersten** vorbestimmten **Arbeitsposition** (301) und einer zweiten vorbestimmten **Arbeitsposition** (302) rotierbar ist, wobei in der ersten Arbeitsposition (301) der **erste Elektrodenbereich** (320; 321) des ersten Maulteils (30) dem zweiten Maulteil (40) zugewandt ist, und wobei in der zweiten Arbeitsposition (302) der **zweite Elektrodenbereich** (340; 322) des ersten Maulteils (30) dem zweiten Maulteil (40) zugewandt ist, wobei der erste Elektrodenbereich (320) durch eine **erste Elektrode** (32) und der zweite Elektrodenbereich (340) durch eine **zweite Elektrode** (34) gebildet sind, **dadurch gekennzeichnet, dass** die erste Elektrode (32) und die zweite Elektrode (34) elektrisch voneinander isoliert sind, und
ferner mit:
einer elektrischen **Kontakteinrichtung** (62) zur elektrischen Kontaktierung lediglich der ersten Elektrode (32), wenn das erste Maulteil (30) sich in der ersten Arbeitsposition (301) befindet, und zur elektrischen Kontaktierung lediglich der zweiten Elektrode (34), wenn das erste Maulteil (30) sich in der zweiten Arbeitsposition (302) befindet.

2. Elektrochirurgisches Instrument (10) nach einem der vorangehenden Ansprüche, bei dem der erste Elektrodenbereich (320; 321) eine stärkere Krümmung aufweist als der zweite Elektrodenbereich (340; 322).

3. Elektrochirurgisches Instrument (10) nach einem der vorangehenden Ansprüche, bei dem das zweite Maulteil (40) einen Elektrodenbereich (420) aufweist, der dem ersten Maulteil (30) zugewandt ist.

4. Elektrochirurgisches Instrument (10) nach einem der vorangehenden Ansprüche, ferner mit:
einem Schaft (15), an dessen distalem Ende (14) die Maulteile (30, 40) angeordnet sind;
einer Handhabungseinrichtung (50) mit einer **Rotationseinrichtung** (58) am proximalen Ende (16) des Schafts (15), wobei die Rotationseinrichtung (58) zur manuellen Betätigung ausgebildet ist;
einer **Übertragungseinrichtung** (22), welche die Rotationseinrichtung (58) mit dem ersten Maulteil (30) mechanisch koppelt, zur Übertragung zumindest entweder eines Drehmoments oder einer Kraft von der Rotationseinrichtung (58) zum ersten Maulteil (30), um das erste Maulteil (30) zwischen seiner ersten Arbeitsposition (301) und seiner zweiten Arbeitsposition (302) zu rotieren.

5. Elektrochirurgisches Instrument (10) nach dem vorangehenden Anspruch, bei dem das erste Maulteil (30) zusammen mit der Übertragungseinrichtung (22) nach distal aus dem Schaft (15) entnehmbar ist.

6. Elektrochirurgisches Instrument (10) nach einem der vorangehenden Ansprüche, das so ausgebildet ist, dass das erste Maulteil (30) nach distal abnehmbar ist.

7. Elektrochirurgisches Instrument (10) nach einem der vorangehenden Ansprüche, bei dem zumindest entweder der erste Elektrodenbereich (320; 321) des ersten Maulteils (30) oder der zweite Elektrodenbereich (340; 322) des ersten Maulteils (30) oder ein dem ersten Maulteil (30) zugewandter Oberflächenbereich (46) des zweiten Maulteils (40) in der Nähe der Schwenkachse (48) in Längsrichtung **konkav** ist.

## Claims

1. Electrosurgical instrument (10), having:
a first jaw part (30) with a first electrode area (320; 321) and a second electrode area (340; 322);
a second jaw part (40),
wherein at least either the first jaw part (30) or the second jaw part (40) is pivotable about a pivot axis (48), such that the jaw parts (30, 40) can be moved towards each other or away from each other,
wherein the first jaw part (30) is rotatable, relative to the second jaw part (40), about a rotation axis (38) between a first predetermined working position (301) and a second predetermined working position (302), wherein in the first working position (301) the first electrode area (320; 321) of the first jaw part (30) faces towards the second jaw part (40), and wherein in the second working position (302) the second electrode area (340; 322) of the first jaw part (30) faces towards the second jaw part (40), wherein the first electrode area (320) is formed by a first electrode (32), and the second electrode area (340) is formed by a second electrode (34), **characterized in that** the first electrode (32) and the second electrode (34) are electrically insulated from each other, and
also having:
an electrical contact device (62) for electrical contacting only of the first electrode (32) when the first jaw part (30) is located in the first working position (301), and for electrical contacting only of the second electrode (34) when the first jaw part (30) is located in the second working position (302).

2. Electrosurgical instrument (10) according to the preceding claim, in which the first electrode area (320; 321) has a stronger curvature than the second electrode area (340; 322).

3. Electrosurgical instrument (10) according to one of the preceding claims, in which the second jaw part (40) has an electrode area (420), which faces towards the first jaw part (30).

4. Electrosurgical instrument (10) according to one of the preceding claims, also having:
a shank (15), on the distal end (14) of which the jaw parts (30, 40) are arranged;
a handling device (50) with a rotation device (58) at the proximal end (16) of the shank (15), wherein the rotation device (58) is designed for manual actuation;
a transmission device (22), which mechanically couples the rotation device (58) to the first jaw part (30), for transmitting at least either a torque or a force from the rotation device (58) to the first jaw part (30) in order to rotate the first jaw part (30) between its first working position (301) and its second working position (302).

5. Electrosurgical instrument (10) according to the preceding claim, in which the first jaw part (30) is removable from the shank (15) in the distal direction together with the transmission device (22).

6. Electrosurgical instrument (10) according to one of the preceding claims, which instrument (10) is designed such that the first jaw part (30) is detachable in the distal direction.

7. Electrosurgical instrument (10) according to one of the preceding claims, in which at least either the first electrode area (320; 321) of the first jaw part (30) or the second electrode area (340; 322) of the first jaw part (30), or a surface area (46) of the second jaw part (40) facing towards the first jaw part (30), is concave in the longitudinal direction in the vicinity of the pivot axis (48).

## Revendications

1. Instrument électrochirurgical (10) présentant
une première partie d'embouchure (30) dotée d'une première zone d'électrode (320; 321) et d'une deuxième zone d'électrode (340; 322),
une deuxième partie d'embouchure (40),
la première partie d'embouchure (30) et/ou la deuxième partie d'embouchure (40) pouvant pivoter autour d'un axe de pivotement (48) de telle sorte que les parties d'embouchure (30, 40) puissent être approchées ou éloignées l'une de l'autre,
la première partie d'embouchure (30) pouvant tourner par rapport à la deuxième partie d'embouchure (40) autour d'un axe de rotation (38) entre une première position prédéterminée de travail (301) et une deuxième position prédéterminée de travail (302),
la première zone d'électrode (320; 321) de la première partie d'embouchure (30) étant tournée vers la deuxième partie d'embouchure (40) dans la première position de travail (301) et la deuxième zone d'électrode (340; 322) de la première partie d'embouchure (30) étant tournée vers la deuxième partie d'embouchure (40) dans la deuxième position de travail (302),
la première zone d'électrode (320) étant formée par une première électrode (32) et la deuxième zone d'électrode (340) par une deuxième électrode (34), la première électrode (32) et la deuxième électrode (34) étant électriquement isolées l'une de l'autre,
et présentant en outre
un dispositif (62) de contact électrique qui assure le contact électrique uniquement avec la première électrode (32) lorsque la première partie d'embouchure (30) se trouve dans la première position de travail (301) et qui assure le contact électrique uniquement avec la deuxième électrode (34) lorsque la première partie d'embouchure (30) se trouve dans la deuxième position de travail (302).

2. Instrument électrochirurgical (10) selon la revendication précédente, dans lequel la première zone d'électrode (320; 321) présente une courbure plus forte que la deuxième zone d'électrode (340; 322).

3. Instrument électrochirurgical (10) selon l'une des revendications précédentes, dans lequel la deuxième partie d'électrode (40) présente une zone d'électrode (420) tournée vers la première partie d'embouchure (30).

4. Instrument électrochirurgical (10) selon l'une des revendications précédentes, doté en outre de :
un arbre (15) à l'extrémité distale (14) duquel les parties d'embouchure (30, 40) sont disposées,
un dispositif de manipulation (50) doté d'un dispositif de rotation (58) à l'extrémité proximale (16) de la tige (15), le dispositif de rotation (58) étant configuré pour être actionné manuellement,
un dispositif de transmission (22) qui accouple mécaniquement le dispositif de rotation (58) et la première partie d'embouchure (30) pour transmettre au moins un couple de rotation ou une force par le dispositif de rotation (58) vers la première partie d'embouchure (30) pour faire tourner la première partie d'embouchure (30) entre sa première position de travail (301) et sa deuxième position de travail (302).

5. Instrument électrochirurgical (10) selon la revendication précédente, dans lequel la première partie d'embouchure (30) peut être enlevée distalement de la tige (15) en même temps que le dispositif de transmission (22).

6. Instrument électrochirurgical (10) selon l'une des revendications précédentes, configuré de telle sorte que la première partie d'embouchure (30) puisse être enlevée distalement.

7. Instrument électrochirurgical (10) selon l'une des revendications précédentes, dans lequel au moins la première zone d'électrode (320; 321) de la première partie d'embouchure (30), la deuxième zone d'électrode (340; 322) de la première partie d'embouchure (30) ou une partie (46) de la surface de la deuxième partie d'embouchure (40) tournée vers la première partie d'embouchure (30) sont concaves dans la direction longitudinale à proximité de l'axe de pivotement (48).
